# EUROPEAN PATENT APPLICATION

(11) **EP 4 239 337 A1**
(43) Date of publication of application: **06.09.2023**
(21) Application number: 21886637.4
(22) Date of filing: 15.10.2021
(51) Int. Cl.: G01N 33/68, C12Q 1/6883

(54) **METHOD FOR DETERMINING MORTALITY RISK OF INFECTIOUS INFLAMMATORY DISEASE ON BASIS OF CONCENTRATION OF WARS AND CYTOKINE**

(30) Priority: 27.10.2020 KR 20200140370
(71) Applicant: MirimGENE Co., Ltd., 21999 Incheon (KR)
(72) Inventor: JIN, Mirim, Seoul 05649 (KR); CHOI, Yun Hui, Incheon 21910 (KR)
(74) Representative: V.O.
(86) International application number: PCT/KR2021/014300
(87) International publication number: WO 2022/092643

(57) **Abstract**

The present disclosure relates to a method for determining the mortality risk of an infectious inflammatory disease on the basis of the concentrations of tryptophanyl-tRNA synthetase (WARS) and cytokines. The method for determining the mortality risk of the present disclosure can effectively identify patients who are at high risk of mortality due to an infectious inflammatory disease within a short period of time and, as such, can be very helpful for performing timely therapy.

## Description

### [Technical Field]

The present disclosure relates to a method for determining the mortality risk of an infectious inflammatory disease based on the concentrations of WARS and cytokines.

### [Background Art]

Aminoacyl-tRNA synthetase (ARS) is an enzyme that attaches an amino acid specifically onto a tRNA and plays a critical role in the production of proteins. Recently, it has been known that ARS is involved in various biological phenomena such as apoptosis, angiogenesis, inflammatory responses, etc. in addition to its essential functions. Especially, human tryptophanyl-tRNA synthetase 1 (WARS1), which is an aminoacyl-tRNA synthetase that recognizes tryptophan and attaches it to a tRNA for protein synthesis, has been recently known to play a role in the host defense mechanism against infection. The inventors of the present disclosure have previously reported that WARS1 is promptly secreted by monocytes into the extracellular space within several minutes following infection without *de novo* synthesis (Young Ha Ahn. et al. Secreted tryptophanyl-tRNA synthetase as a primary defense system against infection. Nature Microbiology 2: 16191 (2016)).

Functioning as an endogenous ligand of TLR4 and TLR2, the secreted WARS1 induces activation of innate immunity by activating macrophages. In addition, the WARS1 initiates the production of proinflammatory cytokines and chemokines such as TNF-α, IL-6, MIP-1α, IL-8 and IFN-γ and induces the infiltration of neutrophils. Subsequently, the WARS1 is secreted continuously as the expression of the WARS1 gene is activated by IFN-γ. Congruously with these findings, the WARS1 is highly enriched in the blood of septic patients but not in aseptic chronic inflammatory disorders. It is secreted regardless of pathogen types such as gram-positive and negative bacteria, viruses, fungi, etc.

Sepsis is a systemic inflammatory response syndrome occurring as a complication of an infectious disease. If the cause is not diagnosed quickly and accurately in the early stage, it may be life-threatening as it develops into severe sepsis, septic shock, multiple organ dysfunction syndrome (MODS) causing functional disorders of the lung, kidney, liver, circulatory organs, etc., disseminated intravascular coagulation (DIC), acute respiratory distress syndrome (ARDS) or acute kidney injury (AKI).

In Korea, the number of patients who were hospitalized or received treatment as outpatients due to sepsis as one of infectious inflammatory diseases increased from 22,430 in 2004 to 34,371 in 2008 (National Health Insurance Service. Statistics of sepsis. 2009.). The mortality of severe sepsis reaches 30% (Tae Nyoung Chung, et al., Application of Mortality in Emergency Department Sepsis (MEDS) Scoring System in the Evaluation of Suspected Sepsis in an Emergency Department. Journal of the Korean Society of Emergency Medicine 2007; 18(2): 150-57). Augus et al. (Epidemiology of severe sepsis in the United States: analysis of incidence, outcome, and associated costs of care. Crit Care Med 2001; 29: 1303-10) reported that intensive care is necessary for more than half of septic patients. It is not easy to predict the onset of the severe situation that threaten the life of a patient. For this reason, it is also difficult to detect such situation early and perform quick and proper treatment within the golden hour.

In order to reduce the mortality of patients by more accurately predicting the severe situations, it is necessary to develop a method capable of determining or diagnosing the severity of patients quickly and accurately. Korean Patent Registration No. 10-1771697 discloses a method of diagnosing sepsis or septic shock by comparing the expression level of WARS with normal people. However, the patent merely identifies that the level of WARS in dead patients is significantly higher than that of surviving patients and fails to disclose a method for accurately distinguishing a patient group showing a low WARS concentration but having a high risk of mortality (or a patient group showing a high WARS concentration but exhibiting high survivability) through correlation with other cytokines and/or chemokines.

The description given in the Background Art section is provided only to enhance the understanding of the background of the present disclosure and should not be construed as recognizing that it is well known to those having ordinary knowledge in the art.

### [Disclosure]

### [Technical Problem]

The inventors of the present disclosure have made consistent efforts to find out a method for effectively screening patients with high mortality risk using the samples of patients having infectious inflammatory diseases such as sepsis. As a result, they have completed the present disclosure by developing a method for determining patients with high mortality risk with accuracy by measuring the concentrations of WARS and cytokines (e.g., IL-8).

The present disclosure is directed to providing a method for providing information necessary for determining the mortality risk of an infectious inflammatory disease, which includes a step of measuring the concentration of WARS (tryptophanyl-tRNA synthetase) and cytokines in a sample of a subject.

The present disclosure is also directed to providing a kit for determining the mortality risk of an infectious inflammatory disease, which includes an agent for measuring the expression level of the WARS protein or a gene encoding the same gene encoding the same; and an agent for measuring the expression level of a cytokine protein or a gene encoding the same.

Other purposes and advantages of the present disclosure will become more apparent by the following detailed description, claims and drawings.

### [Technical Solution]

In an aspect, the present disclosure provides a method for providing information necessary for determining the mortality risk of an infectious inflammatory disease, which includes a step of measuring the concentrations of WARS (tryptophanyl-tRNA synthetase) and cytokines in a sample of a subject.

The inventors of the present disclosure have made consistent efforts to find out a method for effectively screening patients with high mortality risk using the sample of a patient having an infectious inflammatory disease such as sepsis. As a result, they have completed the present disclosure by developing a method for determining a patient with high mortality risk with accuracy by measuring the concentrations of WARS and cytokines (e.g., IL-8).

In the present specification, the term "WARS" refers to tryptophanyl-tRNA synthetase. It is also known as tryptophan-tRNA ligase, TrpRS, WRS, etc. WARS is an enzyme which mediates the aminoacylation reaction between the amino acid tryptophan and a tRNA. In human, WARS is encoded by the WARS gene. The protein's amino acid sequence and mRNA base sequence are known as Genbank accession number NP_004175.2 (protein) and Genbank accession number NM_004184.3 (mRNA base sequence). WARS has two isoforms: cytoplasmic form (WARS1 or tryptophanyl-tRNA synthetase 1, cytoplasmic) and mitochondrial form (WARS2 or tryptophanyl-tRNA synthetase 2, mitochondrial).

According to a specific exemplary embodiment of the present disclosure, the WARS of the present disclosure is WARS1 (SEQ ID NO 1).

According to a specific exemplary embodiment of the present disclosure, the method further includes a step of providing the sample of the subject.

In the present specification, the term "subject" refers to an individual having an infectious inflammatory disease, suspected to have an infectious inflammatory disease or having the risk of having an infectious inflammatory disease (including an individual exposed to viruses, bacteria, fungi, etc. that cause an infectious inflammatory disease), and includes mammals (dog, cat, cow, horse, etc.) including human, birds, reptiles, amphibians, etc. Specifically, the subject is human.

In the present specification, the term "infectious inflammatory disease" refers to an infectious disease accompanied by inflammation, which is caused by infection by viruses, bacteria, fungi, etc. Specifically, it may be one or more disease selected from a group consisting of pneumonia, phthisis, tuberculosis, sepsis and septic shock, although not being limited thereto.

The sepsis includes early-onset sepsis, severe sepsis, septic shock and multiple organ dysfunction syndrome (MODS), disseminated intravascular coagulation (DIC), acute respiratory distress syndrome (ARDS) or acute kidney injury (AKI) accompanied by sepsis, although not being limited thereto.

According to a specific exemplary embodiment of the present disclosure, the infectious inflammatory disease is sepsis or septic shock.

In the present specification, the term "sample" refers to a biological sample isolated or harvested naturally or artificially from a subject. Specifically, it is a cell, a tissue, blood, whole blood, plasma or serum, although not being limited thereto.

In the present specification, the term "cytokine" refers to a protein immunomodulator secreted from immune cells, and includes a chemokine.

The cytokine includes IL-8, IL-6, MIP-1α, INF-γ, TNF-α, IL-1β, IL-10, etc.

The concentration of the WARS and/or cytokine can be determined by measuring their expression level. The expression level of the WARS and/or cytokine may be the expression level of these proteins or genes encoding the same.

In the present specification, the term "expression" refers to the production of a protein or a nucleic acid in a cell. The term protein is used interchangeably with a polypeptide or a peptide. A polynucleotide or a nucleic acid refers to a single- or double-stranded deoxyribonucleotide (DNA) or ribonucleotide (RNA). Unless specified otherwise, known analogues of naturally occurring nucleotides hybridized with nucleic acids in a manner similar to naturally occurring nucleotides are included. mRNA is an RNA which conveys genetic information (specific base sequence of a gene) specified by an amino acid sequence from a specific gene to a ribosome during protein synthesis.

The expression level of the protein may be detected or measured using an antibody binding specifically to the WARS and/or cytokine protein or a fragment thereof (specifically, an antigen-binding fragment). The expression level of the protein may be measured using a method known in the art without limitation. For example, western blotting, dot blotting, enzyme-linked immunosorbent assay (ELISA), radioimmunoassay (RIA), radial immunodiffusion, Ouchterlony immunodiffusion, rocket immunoelectrophoresis, immunohistochemistry, immunoprecipitation, complement fixation assay, flow cytometry (FACS), protein chip assay, etc. may be used, although not being limited thereto. Specifically, ELISA may be used.

In the present specification, the term "antibody" refers to a molecule having an antigen-binding site for specific binding to an antigen. As used in the present specification, the term includes a full-length antibody, any fragment thereof (i.e., an "antigen-binding moiety") or a single chain thereof. According to an exemplary embodiment of the present disclosure, the "antibody" refers to a glycoprotein including at least two heavy chains (H) and two light chains (L) connected by disulfide bonds or an antigen-binding moiety thereof. According to another exemplary embodiment of the present disclosure, the "antibody" refers to a single-chain antibody including a single variable region (domain), e.g., a V_{H}H domain. Each heavy chain consists of a heavy chain variable region (V_{H}) and a heavy chain constant region. In general, the heavy chain constant region includes three domains, C_{H}1, C_{H}2 and C_{H}3, and each light chain includes a light chain variable region (abbreviated as V_{L}) and a light chain constant region. The light chain constant region includes one domain, i.e., C_{L}.

The V_{H} and V_{L} domains can be further subdivided into hypervariable regions, referred to as complementarity-determining regions (CDRs), which are interposed between more conserved regions called framework regions (FRs). Each V_{H} and V_{L} is composed of three CDRs and four FRs, which are arranged from the amino-terminus to the carboxyl-terminus in the order of FR1, CDR1, FR2, CDR2, FR3, CDR3 and FR4. The variable region of the heavy chain and the light chain includes a binding domain that interacts with an antigen. The constant region of the antibody may mediate the binding of the immunoglobulin to host tissues or factors, including various cells of the immune system (e.g., effector cells) and the first component of the classical complement system (C1q).

In the present specification, the term "Fc" refers to the C-terminal region of the antibody heavy chain which mediates the binding of the immunoglobulin to host tissues or factors, including the FC receptors on various cells of the immune system (e.g., effector cells) and the first component of the classical complement system (C1q). Accordingly, the Fc includes the constant region of the antibody excluding the first constant region immunoglobulin domain (e.g., C_{H}1 or C_{L}). In IgG, IgA and IgD antibody isotypes, the Fc region includes two identical protein fragments derived from the second (C_{H}2) and third (C_{H}3) constant domains of the antibody's two heavy chains. In IgM and IgE, the Fc region includes three heavy chain constant domains (C_{H} domain 2-4) in each polypeptide chain. In the present specification, the Fc may be a native sequence including any allotypic variant or an Fc variant (e.g., non-naturally occurring Fc). In addition, the Fc may be an Fc-containing protein polypeptide such as "Fc region-region containing fusion protein", also called an "Fc fusion protein" (e.g., an antibody or immunoadhesin).

The antibody can be of any type (e.g., IgG, IgE, IgM, IgD, IgA or IgY) or any subclass (e.g., IgG1, IgG2, IgG3 or IgG4 in human, and IgG1, IgG2a, IgG2b or IgG3 in mouse) of immunoglobulin molecules. The immunoglobulin, e.g., IgG1, exists as several allotypes which differ from each other in a few amino acids. The antibody disclosed in the present specification may be derived from any of the commonly known isotypes, classes, subclasses or allotypes. In a specific exemplary embodiment, the antibody presented in the present specification belongs to the IgG1, IgG2, IgG3 or IgG4 subclass or any hybrid thereof. In a specific exemplary embodiment, the antibody belongs to the IgG2, IgG4 or IgG2/IgG4 subclass.

The antibody includes, by way of examples, naturally occurring or non-naturally occurring antibodies; monoclonal or polyclonal antibody; chimeric or humanized antibodies; human or non-human antibodies; wholly synthetic antibodies; single-chain antibody; monospecific antibodies; and multispecific antibodies (including bispecific or trispecific antibodies).

In the present specification, the term "neutralizing antibody" refers to an antibody that defends a cell from a pathogen or infectious particle by neutralizing any effect it has biologically. The neutralizing antibody is a part of the adaptive immune response of against viruses, bacteria, fungi and microbial toxins. The neutralizing antibody achieves immunity by binding to the surface structure of the infectious particle and thereby preventing the infectious antigen from interacting with the host cell.

In the present specification, the term "fragment" is understood to include all forms such as one or more fragment of an antibody having the ability of binding specifically to an antigen or the "one or more fragment of an antibody having the ability of binding specifically to an antigen" bound to another molecule (including a part (e.g., Fc) of the same or different antibody). Examples of the fragment include a monovalent fragment (Fab fragment) consisting of V_{L}, V_{H}, C_{L} and C_{H}1 domains; a bivalent fragment (F(ab')₂ fragment) including two Fab fragments linked by a disulfide bridge at the hinge region; an Fd fragment consisting of V_{H} and C_{H}1 domains; an Fv fragment consisting of the V_{L} and V_{H} domains of a single arm of an antibody and disulfide-linked Fv (sdFv); a dAb fragment consisting of a V_{H} domain; and an isolated complementarity-determining region (CDR) or a combination of two or more isolated CDRs which can be optionally be joined by a linker. Furthermore, V_{L} and V_{H} regions may be joined by a linker so that they are paired as a single protein chain to form a monovalent molecule (single-chain Fv (scFv)). These single-chain antibodies are also included in the antibody fragments. In addition, the antibody or antibody fragment includes tetrameric antibodies including two heavy chain molecules and two light chain molecules; antibody light chain monomers; antibody heavy chain monomers; antibody light chain dimers, antibody heavy chain dimers; intrabodies; monovalent antibodies; camelized antibodies; and single-domain antibodies (sdAbs).

According to a specific exemplary embodiment of the present disclosure, the antibody fragment of the present disclosure is a fragment selected from a group consisting of Fab, F(ab')₂, Fd, sdFv, Fv, dAb, scFv, sdAb and a tetramer or the fragment bound to Fc.

The expression level of a gene encoding the WARS and/or cytokine may be determined by measuring the mRNA level of the protein. The existence and expression level of the mRNA in the sample of a subject may be measured by amplifying the mRNA or cDNA of the WARS and/or cytokine using a primer set or a probe that binds specifically thereto or by hybridizing the same with a probe. For the measurement of the mRNA expression level, conventional methods for identification of expression level may be used without limitation, such as reverse transcription polymerase chain reaction (RT-PCR), competitive RT-PCR, real-time RT-PCR, RNase protection assay (RPA), northern blotting, DNA microarray chip assay, RNA sequencing, NanoString hybridization, in-situ hybridization of tissue sections, etc., although not being limited thereto.

In the present specification, the term "primer" refers to a short single-stranded oligonucleotide acting as a starting point of DNA synthesis. The primer binds specifically to a polynucleotide as a template under the condition of a suitable buffer and temperature. DNA is synthesized as a DNA polymerase links a nucleoside triphosphate having a base complementary to the template DNA to the primer. The primer is generally composed of 15-30 base sequences, and the melting temperature (Tₘ) for binding to the template strand varies depending on the constitution and length of the base. The primer may be easily designed by those skilled in the art referring to the mRNA or cDNA base sequence of the WARS and/or cytokine for use in the determination of the mortality risk of an infectious inflammatory disease.

In the present specification, the term "probe" refers to fragment of a polynucleotide, such as RNA, DNA, etc., capable of binding specifically to the mRNA or cDNA (complementary DNA) of a specific gene and having a length of from several to several hundreds of base pairs. The probe may be labeled to identify the presence or absence, expression level, etc. of the target mRNA or cDNA. For the purpose of the present disclosure, a probe complementary to the WRS mRNA may be used to determine the mortality risk of an infectious inflammatory disease by measuring the expression level of the WARS mRNA through hybridization with a sample of a subject. The selection and hybridization condition of the probe may be selected properly according to a method known in the art.

According to a specific exemplary embodiment of the present disclosure, the method of the present disclosure includes a step of measuring the concentration of WARS (tryptophanyl-tRNA synthetase) and IL-8.

According to a specific exemplary embodiment of the present disclosure, the method of the present disclosure includes a step of identifying whether the concentration of WARS is 100 ng/mL or higher and lower than 200 ng/mL and the concentration of IL-8 is 400 pg/mL or higher.

Information useful for determining as a patient group with high mortality risk may be provided when the concentration of WARS is 100 ng/mL or higher and lower than 200 ng/mL and the concentration of IL-8 is 400 pg/mL or higher, specifically 430 pg/mL or higher, more specifically 440 pg/mL or higher, further more specifically 450 pg/mL or higher, even more specifically 460 pg/mL or higher, most specifically 466.5 pg/mL or higher.

According to a specific exemplary embodiment of the present disclosure, the method of the present disclosure includes a step of determining as a patient group with high mortality risk when the concentration of WARS is 100 ng/mL or higher and lower than 200 ng/mL and the concentration of IL-8 is 466.5 pg/mL or higher.

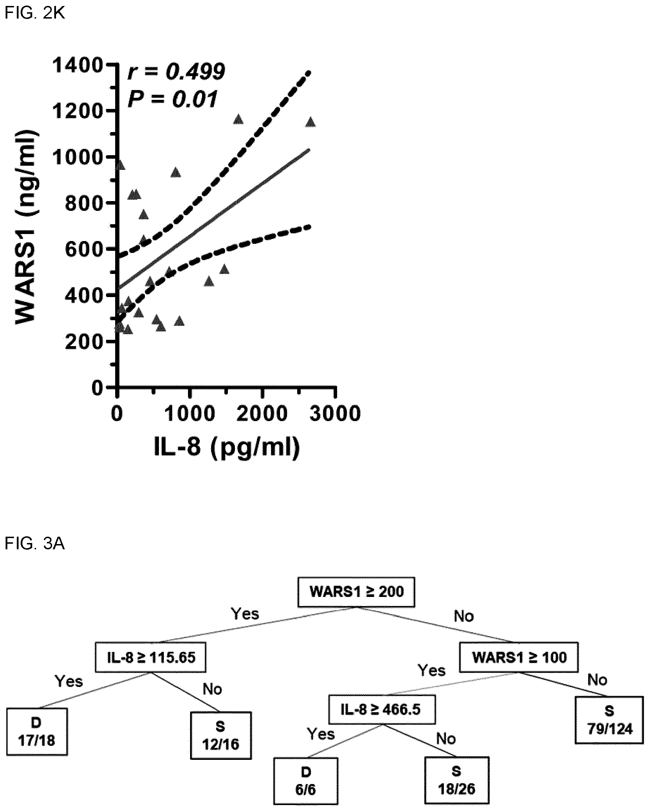

In an example of the present disclosure, all septic patients (6/6) died when the concentration of was 100 ng/mL or higher and lower than 200 ng/mL and the concentration of IL-8 was 466.5 pg/mL or higher (FIG. 3a).

According to a specific exemplary embodiment of the present disclosure, the method of the present disclosure includes a step of identifying whether the concentration of WARS is 200 ng/mL or higher and the concentration of IL-8 is 100 pg/mL or higher.

Information useful for determining as a patient group with high mortality risk may be provided when the concentration of WARS is 200 ng/mL or higher and the concentration of IL-8 is 100 pg/mL or higher, specifically 105 pg/mL or higher, more specifically 110 pg/mL or higher, further more specifically 115 pg/mL or higher, most specifically 115.65 pg/mL or higher.

According to a specific exemplary embodiment of the present disclosure, the method of the present disclosure includes a step of determining as a patient group with high mortality risk when the concentration of WARS is 200 ng/mL or higher and the concentration of IL-8 is 115.65 pg/mL or higher.

In an example of the present disclosure, 94% of septic patients (17/18) died when the concentration of was 200 ng/mL or higher and the concentration of IL-8 was 115.65 pg/mL or higher (FIG. 3a).

According to a specific exemplary embodiment of the present disclosure, the method of the present disclosure includes a step of identifying whether the concentration of WARS is lower than 200 ng/mL and the concentration of IL-6 is 1,000 pg/mL or higher.

Information useful for determining as a patient group with high mortality risk may be provided when the concentration of WARS is lower than 200 ng/mL and the concentration of IL-6 is 1,000 pg/mL or higher, specifically 1,200 pg/mL or higher, more specifically 1,300 pg/mL or higher, further more specifically 1,500 pg/mL or higher, even more specifically 1,600 pg/mL or higher, most specifically 1654.5 pg/mL or higher.

According to a specific exemplary embodiment of the present disclosure, the method of the present disclosure includes a step of determining as a patient group with high mortality risk when the concentration of WARS is lower than 200 ng/mL and the concentration of IL-6 is 1,654.5 pg/mL or higher.

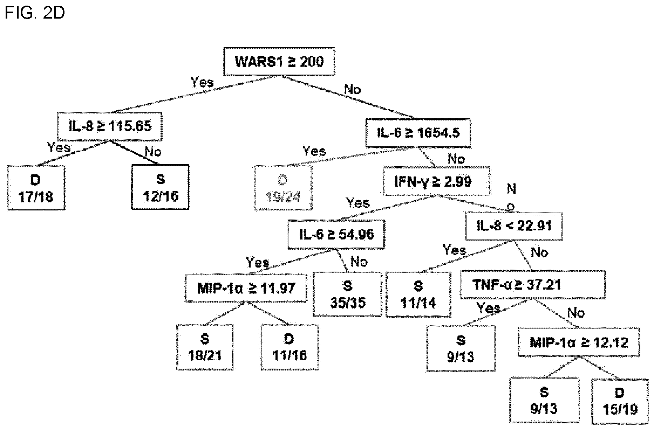

In an example of the present disclosure, 79.2% of septic patients (19/24) died when the concentration of WARS was lower than 200 ng/mL and the concentration of IL-6 was 1654.5 pg/mL or higher (FIG. 2d).

According to a specific exemplary embodiment of the present disclosure, the method of the present disclosure includes a step of further identifying the concentration of one or more cytokine selected from a group consisting of IFN-γ, IL-6, MIP-1α, IL-8 and TNF-α when the concentration of WARS is lower than 200 ng/mL and the concentration of IL-6 is lower than 1,654.5 pg/mL.

When the concentration of WARS is lower than 200 ng/mL and the concentration of IL-6 is lower than 1654.5 pg/mL, the concentration of IFN-γ may be further identified.

According to a specific exemplary embodiment of the present disclosure, the method of the present disclosure includes a step of determining as a patient group with low mortality risk when the concentration of IFN-γ is 2.99 pg/mL or higher and the concentration of IL-6 is lower than 54.96 pg/mL.

In an example of the present disclosure, when the concentration of WARS was lower than 200 ng/mL and the concentration of IL-6 was lower than 1654.5 pg/mL, 100% of septic patients (35/35) survived when the concentration of IFN-γ was 2.99 pg/mL or higher and the concentration of IL-6 was lower than 54.96 pg/mL (FIG. 2d).

According to a specific exemplary embodiment of the present disclosure, the method of the present disclosure includes a step of determining as a patient group with low mortality risk when the concentration of IFN-γ is lower than 2.99 pg/mL and the concentration of IL-8 is lower than 22.91 pg/mL.

In an example of the present disclosure, 78.6% of septic patients (11/14) survived when the concentration of WARS was lower than 200 ng/mL, the concentration of IL-6 was lower than 1654.5 pg/mL, the concentration of IFN-γ was lower than 2.99 pg/mL and the concentration of IL-8 was lower than 22.91 pg/mL (FIG. 2d).

According to a specific exemplary embodiment of the present disclosure, the method of the present disclosure includes a step of determining as a patient group with low mortality risk when the concentration of IFN-γ is lower than 2.99 pg/mL, the concentration of IL-8 is 22.91 pg/mL or higher and the concentration of TNF-α is 37.21 pg/mL or higher.

In an example of the present disclosure, 69.2% of septic patients (9/13) survived when the concentration of WARS was 200 ng/mL, the concentration of IL-6 was lower than 1654.5 pg/mL, the concentration of IFN-γ was lower than 2.99 pg/mL, the concentration of IL-8 was 22.91 pg/mL or higher and the concentration of TNF-α was 37.21 pg/mL (FIG. 2d).

In another aspect, the present disclosure provides a kit for determining the mortality risk of an infectious inflammatory disease, which includes: an agent for measuring the expression level of the WARS protein or a gene encoding the same; and an agent for measuring the expression level of the IL-8 protein or a gene encoding the same.

According to a specific exemplary embodiment of the present disclosure, the cytokine of the present disclosure is IL-8 or IL-6.

The expression level of the protein may be detected or measured using an antibody that binds specifically to the WARS and/or cytokine protein or a fragment thereof (specifically, an antigen-binding fragment).

The expression level of the gene encoding the WARS and/or cytokine may be determined by measuring the mRNA level of the protein. The existence and expression level of the mRNA in the sample of a subject may be measured by amplifying the mRNA or cDNA of the WARS and/or cytokine using a primer set or a probe that binds specifically thereto or by hybridizing the same with a probe.

In the following description of a kit for determining the mortality risk of an infectious inflammatory disease, the matters that have been described above in the description of the method for providing information necessary for determining the mortality risk of an infectious inflammatory disease will be omitted to avoid unnecessary redundancy.

According to a specific exemplary embodiment of the present disclosure, the kit of the present disclosure includes: an agent for measuring the expression level of the WARS protein or a gene encoding the same; and an agent for measuring the expression level of the IL-8 protein or a gene encoding the same.

According to a specific exemplary embodiment of the present disclosure, the kit may be for determining the mortality risk of an infectious inflammatory disease by identifying whether the concentration of WARS is 100 ng/mL or higher and the concentration of IL-8 is 100 pg/mL or higher in the sample of the subject.

According to a specific exemplary embodiment of the present disclosure, the kit further include an instruction about the determination of the mortality risk depending on the correlation between the concentration of WARS and the concentration of IL-8 in a sample.

For example, the instruction may describe the identification of whether the concentration of WARS is 200 ng/mL or higher and the concentration of IL-8 is 100 pg/mL or higher, specifically whether the concentration of WARS is 200 ng/mL or higher and the concentration of IL-8 is 115.65 pg/mL or higher.

For example, the instruction may describe the identification of whether the concentration of WARS is 100 ng/mL or higher and lower than 200 ng/mL and the concentration of IL-8 is 400 pg/mL or higher or the identification of whether the concentration of WARS is 100 ng/mL or higher and lower than 200 ng/mL and the concentration of IL-8 is 466.5 pg/mL or higher.

According to a specific exemplary embodiment of the present disclosure, the kit may further include an agent for measuring the expression level of the IL-6 protein or a gene encoding the same.

### [Advantageous Effects]

The features and advantages of the present disclosure may be summarized as follows:
(i) The present disclosure provides a method for determining the mortality risk of an infectious inflammatory disease based on the concentration of tryptophanyl-tRNA synthetase (WARS) and cytokines.
(ii) The method for determining the mortality risk of the present disclosure can effectively identify patients who are at high risk of mortality due to an infectious inflammatory disease within a short period of time and, as such, can be very helpful for performing timely therapy.

### [Brief Description of Drawings]

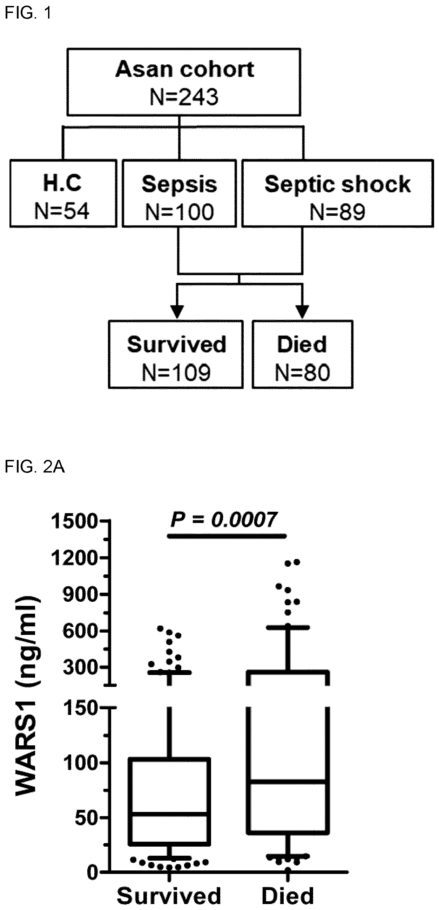

FIG. 1 shows the cohort design for the present disclosure.

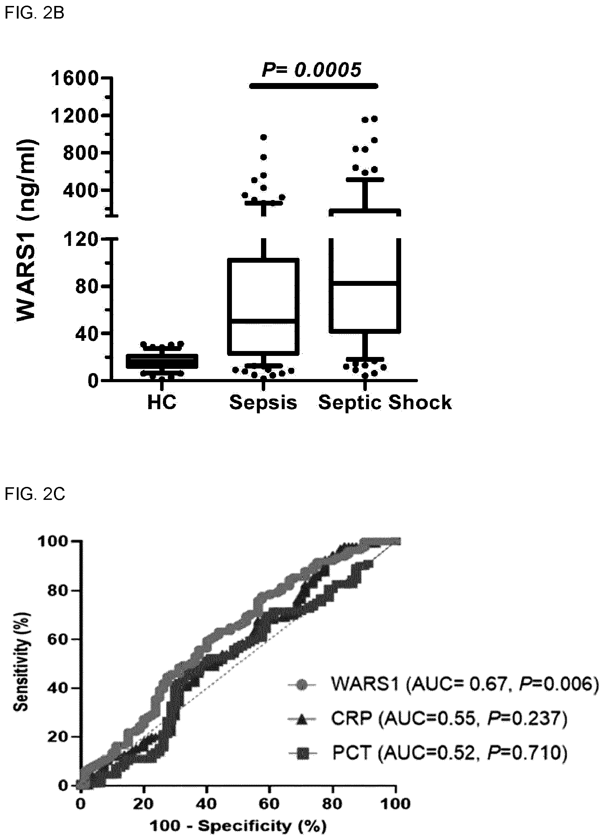

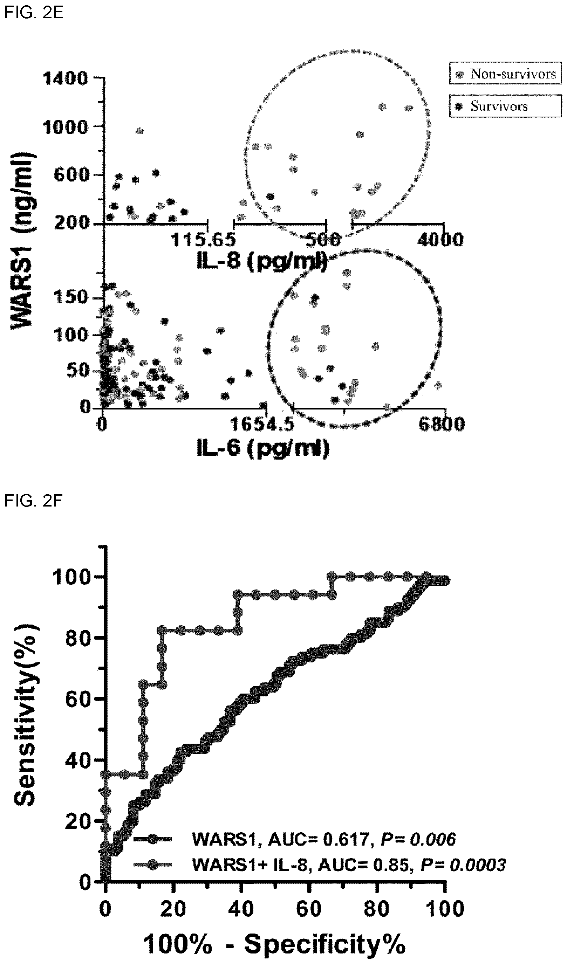

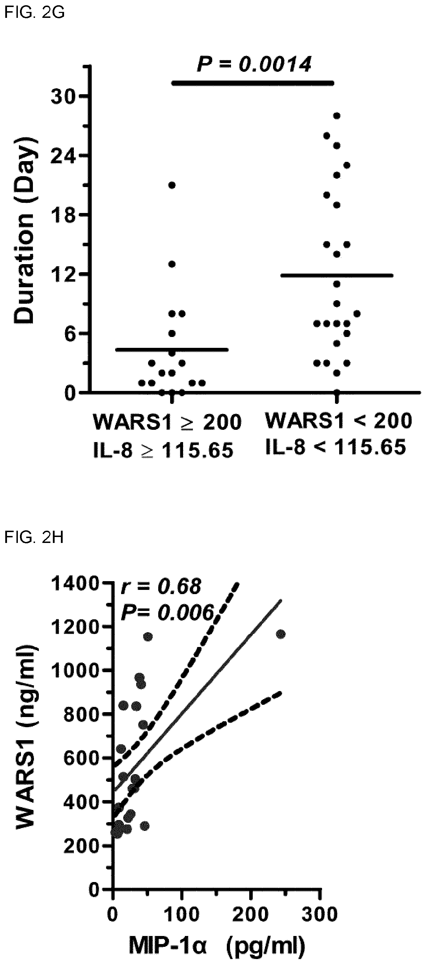

FIGS. 2a-2k show a result of measuring the concentration of WARS1 and cytokines in the samples of survivors and non-survivors. FIG. 2a shows a result of comparing the concentration of WARS1 in survivors and non-survivors, FIG. 2b shows a result of comparing the concentration between a sepsis group and a septic shock group, FIG. 2c shows an AUC analysis result for the survivors and non-survivors, FIG. 2d shows a decision tree obtained using the R2 software from the concentration of WARS1, proinflammatory cytokines and chemokines, FIG. 2e shows the distribution of the concentration of IL-8 and IL-6 based on WARS1, FIG. 2f shows an AUC analysis result for IL-8 and WARS1, FIG. 2g compares days of survival depending on the concentration of WARS1 and IL-8, and FIG. 2h-2k show the correlation and significance of proinflammatory cytokines and chemokines in non-survivors (WARS1 200 ng/mL, IL-8 115.65 pg/mL or higher).

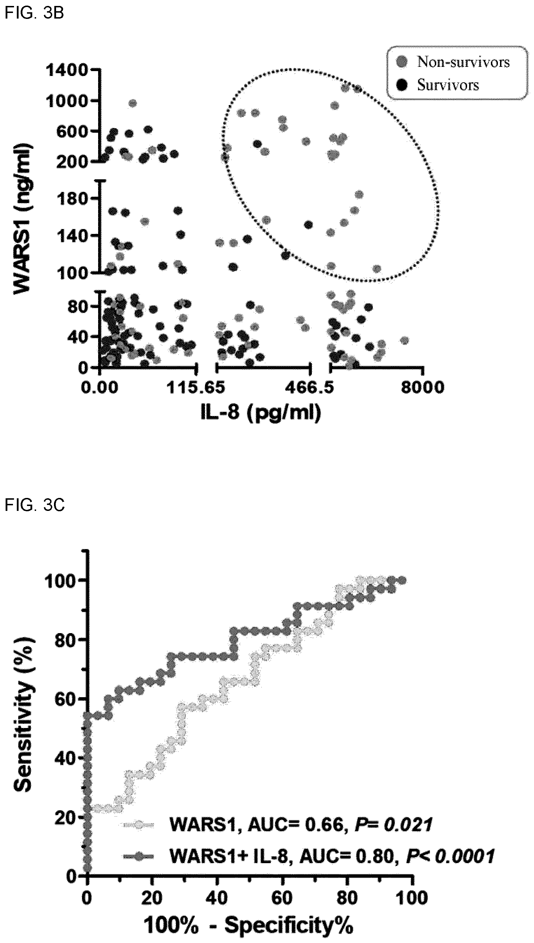

FIGS. 3a-3c show a result of analyzing the correlation of WARS1 and IL-8 in survivors and non-survivors. FIG. 3a shows a decision tree obtained from analysis of WARS1 and IL-8 only, FIG. 3b shows the distribution of the concentration of IL-8 based on WARS1, and FIG. 3c shows an AUC analysis result for IL-8 and WARS1.

### [Best Mode]

Hereinafter, the present disclosure will be described more specifically through examples. The following examples are provided only to describe the present disclosure more specifically and it will be obvious to those having ordinary knowledge in the art that the scope of the present disclosure is not limited by the examples.

### Examples

### Example 1. Cohort design

In order to investigate whether the death caused by sepsis is correlated with the high concentration of WARS1, target patients were selected based on the concentration of WARS1. Plasma samples were acquired from Asan Hospital. The samples had been taken from the patients within 24 hours after hospitalization. The cohort consisted of a total of 243 patients (FIG. 1), with 54 patients for an H.C group (normal group), 100 patients for a sepsis group and 89 patients for a septic shock group.

### Example 2. Comparison of concentration of WARS1, cytokines and chemokines in survivors and non-survivors

The difference in the level of WARS1 and proinflammatory cytokines and chemokines such as IL-1β, IL-6, IL-8, IFN-γ, MIP-1α and TNF-α in the plasma of survivors and non-survivors was measured. The level of IL-1β, IL-6, IL-8, IFN-γ, MIP-1α and TNF-α was measured by multiplex assay (Millipore), and the level of WARS1 was measured by ELISA using an anti-WARS1 antibody (Abiotech). The measurement was performed according to the manufactures' instructions.

WARS1 was significantly increased in the non-survivors as compared to the survivors (FIG. 2a), and was significantly increased in the septic shock group as compared to the sepsis group (FIG. 2b). As a result of AUC analysis for WARS1, procalcitonin (PCT) and C-reactive protein (CRP) as biomarkers, there was no significant difference in the level of PCT (p = 0.7112) and CRP (p = 0.2380) between the non-survivors and the survivors (FIG. 2c). Only WARS1 showed the significant ability of predicting mortality (p = 0.006).

A decision tree (DT) was obtained by analyzing and classifying the concentration of cytokines and chemokines in the plasma of cohort patients depending on the concentration of WARS1 through "rpart" package analysis of the R software (version 3.5.1). The WARS1 concentration of 200 ng/mL was set as a first classification tree root. In the first group, 17 out of 18 septic patients (94.4%) whose IL-8 level was 115.65 pg/mL or higher died. IL-6 was selected as a second root for the patients whose WARS1 level of 200 ng/mL or smaller. 19 out of 24 patients (79.2%) whose IL-6 level was 1654.5 pg/mL or higher died (FIG. 2d). The DT showed a sensitivity of 77.5% and a specificity of 86.2% (FIG. 2e). Non-survivors could be distinguished significantly from the septic patients (p = 0.0003) using the AUC with the WARS1 level of 200 ng/mL or higher and the IL-8 level of 115.65 pg/mL or higher (FIG. 2f).

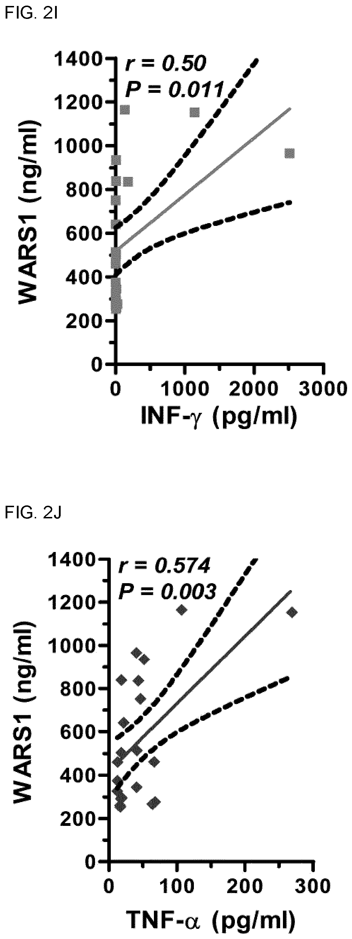

Average days of survival was about 4.3 days for the first group, which was remarkably shorter than that of the non-survivors whose WARS1 level was lower than 200 ng/mL and the IL-8 level was lower than 115.65 pg/mL (FIG. 2g). The first group showed positive correlations between WARS1 and MIP-1α (p = 0.006), IFN-γ (p = 0.011), TNF-α (p = 0.003) and IL-8 (p = 0.01) (FIGS. 2 h-2k). As a result of comparing the level of MIP-1α, INF-γ, TNF-α, IL-6, IL-1β, IL-8 and IL-10 for the normal group, the sepsis group and the septic shock group, it was confirmed that MIP-1α (p < 0.0001), TNF-α (p < 0.0001), IL-6 (p < 0.0001), IL-8 (p < 0.0001) and IL-10 (p < 0.0001) were increased significantly in the septic shock group. In addition, IFN-γ, TNF-α, IL-6, IL-1β, IL-6 and IL-10, except MIP-1α, were increased relatively in the non-survivors as compared to the survivors, and IL-6 (p < 0.0001), IL-8 (p < 0.0001) and IL-10 (p < 0.0001) were increased significantly.

### Example 3. Analysis of correlation of WARS1 and IL-8 in survivors and non-survivors

A decision tree (DT) was obtained for the concentration of WARS1 and IL-8 only through "rpart" package analysis of the R software (version 3.5.1). The WARS1 concentration of 200 ng/mL was set as a classification tree root (100 ng/mL ≤ WARS1 < 200 ng/mL). In the first group, 17 out of 18 septic patients whose WARS1 level was 200 ng/mL or higher and IL-8 level was 115.65 pg/mL or higher died. For the septic patients with 100 ng/mL ≤ WARS1 < 200 ng/mL and IL-8 level of 466.5 ng/mL or higher, all the 6 patients died (FIG. 3a). 24 of the 80 dead patients could be targeted with the DT, and the septic patients could be distinguished specifically as non-survivors with an AUC of 0.80 for WARS1 and IL-8 (p = 0.007) (FIGS. 3b and 3c).

Although the examples of the present disclosure have been described above, those having ordinary knowledge in the art can change and modify the present disclosure variously through addition, change or deletion without departing from the scope of the present disclosure defined by the appended claims.

## Claims

1. A method for providing information necessary for determining the mortality risk of an infectious inflammatory disease, comprising:
(a) a step of providing a sample of a subject;
(b) a step of measuring the concentrations of WARS (tryptophanyl-tRNA synthetase) and IL-8 in the sample; and
(c) a step of identifying whether the concentration of WARS is 100 ng/mL or higher and the concentration of IL-8 is 100 pg/mL or higher.

2. A method for providing information necessary for determining the mortality risk of an infectious inflammatory disease, comprising:
(a) a step of providing a sample of a subject;
(b) a step of measuring the concentrations of WARS (tryptophanyl-tRNA synthetase) and IL-6 in the sample; and
(c) a step of identifying whether the concentration of WARS is lower than 200 ng/mL and the concentration of IL-6 is 1,000 pg/mL or higher.

3. The method for providing information according to claim 1 or 2, wherein the infection is infection by one or more selected from a group consisting of viruses, bacteria and fungi.

4. The method for providing information according to claim 1 or 2, wherein the infectious inflammatory disease is one or more disease selected from a group consisting of pneumonia, phthisis, tuberculosis, sepsis and septic shock.

5. The method for providing information according to claim 4, wherein the infectious inflammatory disease is sepsis or septic shock.

6. The method for providing information according to claim 1 or 2, wherein the sample is blood, plasma or serum.

7. The method for providing information according to claim 1, wherein, in the step (c), it is identified whether the concentration of WARS is 100 ng/mL or higher and lower than 200 ng/mL and the concentration of IL-8 is 400 pg/mL or higher.

8. The method for providing information according to claim 7, wherein the subject is determined as a patient group with high mortality risk if the concentration of WARS is 100 ng/mL or higher 200 ng/mL and the concentration of IL-8 is 466.5 pg/mL or higher.

9. The method for providing information according to claim 1, wherein, in the step (c), it is identified whether the concentration of WARS is 200 ng/mL or higher and the concentration of IL-8 is 100 pg/mL or higher.

10. The method for providing information according to claim 9, wherein the subject is determined as a patient group with high mortality risk if the concentration of WARS is 200 ng/mL or higher and the concentration of IL-8 is 115.65 pg/mL or higher.

11. The method for providing information according to claim 2, wherein, in the step (c), it is identified whether the concentration of WARS is lower than 200 ng/mL and the concentration of IL-6 is 1,600 pg/mL or higher.

12. The method for providing information according to claim 11, wherein the subject is determined as a patient group with high mortality risk if the concentration of WARS is lower than 200 ng/mL and the concentration of IL-6 is 1,654.5 pg/mL or higher.

13. The method for providing information according to claim 2, which further comprises a step (step (d)) of identifying the concentration of one or more cytokine selected from a group consisting of IFN-γ, IL-6, MIP-1α, IL-8 and TNF-α when the concentration of WARS is lower than 200 ng/mL and the concentration of IL-6 is lower than 1,654.5 pg/mL.

14. The method for providing information according to claim 13, wherein the subject is determined as a patient group with low mortality risk if the concentration of IFN-γ is 2.99 pg/mL or higher and the concentration of IL-6 is lower than 54.96 pg/mL.

15. The method for providing information according to claim 13, wherein the subject is determined as a patient group with low mortality risk if the concentration of IFN-γ is lower than 2.99 pg/mL and the concentration of IL-8 is lower than 22.91 pg/mL.

16. The method for providing information according to claim 13, wherein the subject is determined as a patient group with low mortality risk if the concentration of IFN-γ is lower than 2.99 pg/mL, the concentration of IL-8 is 22.91 pg/mL or higher and the concentration of TNF-α is 37.21 pg/mL or higher.

17. A kit for determining the mortality risk of an infectious inflammatory disease, comprising: an agent for measuring the expression level of the WARS protein or a gene encoding the same; and an agent for measuring the expression level of the IL-8 protein or a gene encoding the same.

18. The kit for determining the mortality risk according to claim 17, wherein the kit further comprises an agent for measuring the expression level of the IL-6 protein or a gene encoding the same.

19. The kit for determining the mortality risk according to claim 17, wherein the kit determines the mortality risk of an infectious inflammatory disease by identifying whether the concentration of WARS is 100 ng/mL or higher and the concentration of IL-8 is 100 pg/mL or higher in a sample of a subject.

20. The kit for determining the mortality risk according to claim 17, wherein the kit further comprises an instruction about the determination of the mortality risk depending on the correlation between the concentration of WARS and the concentration of IL-8 in a sample.

21. The kit for determining the mortality risk according to claim 20, wherein the instruction describes the identification of whether the concentration of WARS is 100 ng/mL or higher and lower than 200 ng/mL and the concentration of IL-8 is 400 pg/mL or higher or the identification of whether the concentration of WARS is 100 ng/mL or higher and lower than 200 ng/mL and the concentration of IL-8 is 466.5 pg/mL or higher.
